# EUROPEAN PATENT APPLICATION

(11) **EP 3 741 867 A1**
(43) Date of publication of application: **25.11.2020**
(21) Application number: 19176537.9
(22) Date of filing: 24.05.2019
(51) Int. Cl.: C12Q 1/34, C12Q 1/44

(54) **SULFATASE ACTIVITY ASSAY**

(71) Applicant: Swedish Orphan Biovitrum AB, 112 76 Stockholm (SE)
(72) Inventor: SU, Chao, 112 76 Stockholm (SE)
(74) Representative: AWA Sweden AB

(57) **Abstract**

The present invention provides improved methods for analysing sulphate, a product of the enzymatic activity of a sulfohydrolase. A method encompassed by the present invention comprises a step of colorimetric analysis, said colorimetric analysis comprising colorimetrically measuring the amount of a barium-rhodizonate complex in the presence of any sulphate ions that have been enzymatically removed from a substrate by said sulfohydrolase, said substrate containing at least one sulphur-heteroatom bond and said amount of barium-rhodizonate complex being inversely proportional to the enzymatic activity of said sulfohydrolase, wherein said barium-rhodizonate complex during said step of measuring is present in a solution comprising methanol and at least one solvent selected from the group of an alcohol with 2-6 carbons, an ether alcohol with 4-6 carbons, a heterocycle with at least one heteroatom and a polar aprotic solvent.

## Description

### Technical Field

The present disclosure relates to methods of analyzing an amount of free sulphate, comprising a step of colorimetric analysis. Such methods can also be employed for analysis of enzymatic activity of a sulfohydrolase. The present disclosure furthermore relates to methods employing a step of colorimetric analysis and to use of a solution for colorimetric analysis.

### Background

Sulfatases (EC 3.1.6) and sulfamidases (EC 3.10) are esterases acting on sulphur-heteroatom bonds to release sulphate. Sulfamidase (EC 3.10.1.1) with the systematic name N-sulfo-D-glucoamine sulfohydrolase (also known as sulfamate sulfohydrolase and heparin N-sulfatase) belongs to the sulfatase family of enzymes. Sulfamidase has a native molecular weight of approximately 120 kDa and a subunit size of 56 kDa, indicating the native molecule is a dimer. The gene encoding sulfamidase is deficient in patients suffering from mucopolysaccharidosis (MPS) type IIIA, an inherited lysosomal storage disease. This causes accumulation of heparan sulphate in the lysosomes resulting in degeneration of the central nervous system commencing during early childhood.

In a variety of disease fields, the missing function caused by a mutated or missing protein may be restored by administration, and thus replacement of, the mutated/missing protein with a protein from a heterologous source. This principle is called enzyme replacement therapy (ERT). In the field of lysosomal storage diseases, storage can be reduced by administration of a lysosomal enzyme from a heterologous source. In the event of a promising ERT drug candidate for patients with MPS IIIA, an enzymatic characterization of activity level is of great interest.

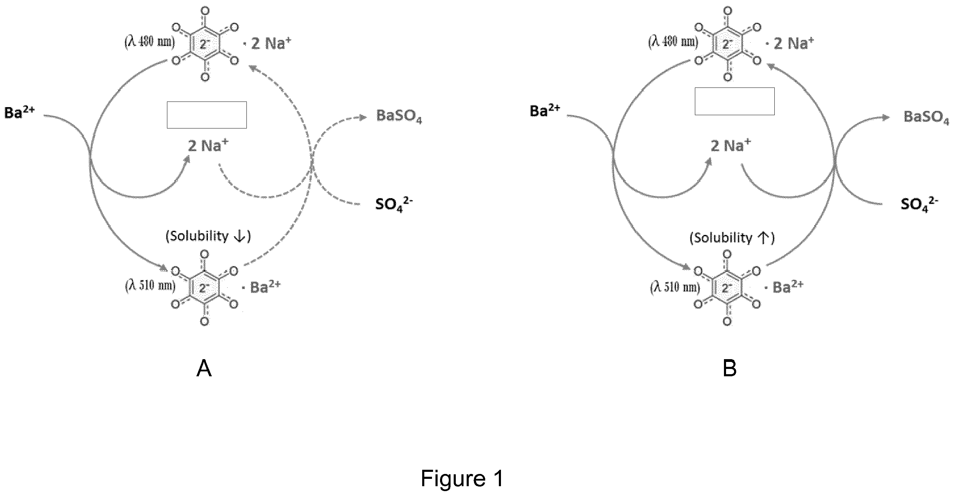

Rhodizonates have been used as qualitative dye reagents for barium, lead and other metals [Chalmers & Telling, Microchimica Acta 55:1126-1135 (1967)]. Rhodizonate forms a complex with barium ions that can be quantified spectrophotometrically. Sulphate reduces the amount of available barium by forming barium sulphate, thereby the amount of sulphate released is inversely proportional to the absorbance of barium rhodizonate. In this way, direct quantification of sulphate anion is possible, which can be represented by the scheme represented in Figure 1A.

In 1971, Terho and Hartiala disclosed the colorimetric method of Barium-rhodizonate, using ethanol as solvent, for measuring sulphate content of glycosaminoglycans [Terho & Hartiala, Anal Biochem 41:471-476 (1971)]. There were several follow-up publications until the early of 1980s, for example, in determining urinary sulphate ion [Swaroop, Clinica Chimica Acta 46:333-336 (1973)], sulphate ion released by iduronate-2-sulfatase [Ginsberg & Ferrante, Biochem Med 17:80-86 (1977)], activity of aryl sulfatase A [Waheed & Etten, Anal Biochem 89:550-560 (1978)], and inorganic sulphate of pyrolytic carbohydrates [Silvestri et al, Anal Biochem 123:303-309 (1982)].

A common problem recognized with this method was that the chromogen barium-rhodizonate complex formed caused a visible precipitate within 30 min. Another common observation was that the fresh prepared rhodizonate reagent needs to mellow out before use in order to establish a calibration linearity. The original procedure described that the reagent can be used after 30 min of preparing [Chalmers & Telling, *supra,* Terho & Hartiala, *supra*], while the follow-up authors said to use it as soon as possible after 30 min [Swaroop, Clinica Chimica Acta 46:333-336 (1973)], to rest overnight at room temperature [Ginsberg & Ferrante, Biochem Med 17:80-86, (1977)], or to stand for 2 h [Waheed & Etten, Anal Biochem 89:550-560 (1978)]. More illustrative is that the reagent requires to expose to light at room temperature for approximately 15 min to achieve a linear standard curve, which is what they called a "curing" step [Silvestri et al, Anal Biochem 123:303-309 (1982)]. Since 1982, no further publications on the subject have followed.

In the light of the above, there is a need in the art for methods of analyzing sulphate, for example to determine the enzymatic activity of a sulfohydrolase.

### Disclosure of the invention

It is an object of the present invention to overcome the problems in the prior art, and to provide improved means for direct analysis of the activity of a sulfohydrolase.

In addition, another object is to provide means for analyzing activity of a sulfohydrolase in a method of manufacturing a sulfohydrolase.

Another object is to provide improved barium-rhodizonate solutions and use of such solutions.

These and other objects, which will be apparent to a skilled person from the present disclosure, are achieved by the different aspects of the invention as defined in the appended claims and as generally disclosed herein.

In a first aspect of the present invention, there is provided a method of analyzing enzymatic activity of a sulfohydrolase, comprising a step of colorimetric analysis wherein the colorimetric analysis comprises: colorimetrically measuring the amount of a barium-rhodizonate complex in the presence of any sulphate ions that have been enzymatically removed from a substrate by a sulfohydrolase. The substrate contains at least one sulphur-heteroatom bond. The amount of barium-rhodizonate complex is inversely proportional to the enzymatic activity of the sulfohydrolase. During the step of colorimetrically measuring, the barium-rhodizonate complex is present in a solution comprising methanol and at least one solvent selected from the group of an alcohol with 2-6 carbons, an ether alcohol with 4-6 carbons, a heterocycle with at least one heteroatom and a polar aprotic solvent.

It has surprisingly been found that the method as defined above provides an excellent assay for direct measurement of the activity of a sulfohydrolase. Solvation of the reactants and products during the reaction is a prerequisite for accurate spectrophotometric quantification. The first aspect of the invention is based on the insight that by introducing a solution comprising methanol and at least one solvent selected from the group of an alcohol with 2-6 carbons, an ether alcohol with 4-6 carbons, a heterocycle with at least one heteroatom and a polar aprotic solvent, the barium-rhodizonate complex can be solvated. This method thus overcomes the problems of prior art methods, where the barium-rhodizonate complex has been found to form a visible precipitate within 30 minutes. It has been hypothesized that non-symmetric species, such as geminal diols [C₆O₄(OH)₄²⁻] could be formed during the displacement of sodium ion with barium ion via a hydration mechanism following up a slow isomerization whose rearrangement, from an *ortho*-dihyrate form to a para-dihydrate form, might cause precipitation with the barium ion (Figure 1A). In comparison, the method according to present invention can provide a stable reading frame more than 2 h due to improved dyeing durability. It is hypothesized that in the method according to the present invention, hydration mechanisms are prevented or depressed by dehydration (Figure 1B). Differences between the prior art and an embodiment of the present invention are illustrated in Figure 1A and B.

The solution is understood to be any solution in which a barium-rhodizonate complex is at least partly dissolved. In some embodiments, the solution may be methanol alone. The solution may be a mixture of at least two solvents; methanol and at least one co-solvent.

The method of analyzing the enzymatic activity of a sulfohydrolase as disclosed herein provides for direct measurement of enzymatic activity. Where possible, direct measurement of enzymatic activity is often preferred over indirect measurement, an example of the latter being enzyme-coupled assays. Direct measurement provides for higher accuracy and ensures a solitary enzyme kinetics, while indirect measurement is not always valid for - enzyme kinetics and characterization. Solitary enzyme kinetics can provide for determination of kinetic parameters such as *V*ₘₐₓ, *K*ₘ and *k*_{cat}, which is of importance for therapeutic enzymes, as *K*ₘ and *k*_{cat} measurements using a physiologically relevant substrate normally have to be included in the release and stability program of Phase III studies.

Furthermore, the prior art methods seemingly required curing of the fresh prepared rhodizonate reagent before use. For example, prior art discloses that the reagent can be used after 30 minutes of preparing [Terho & Hartiala, *supra*]. The method according to some embodiments solves this problem in the prior art by providing a method in which curing can be excluded.

The present inventors have surprisingly found that, by using at least one solvent selected from the group of an alcohol with 2-6 carbons, an ether alcohol with 4-6 carbons, a heterocycle with at least one heteroatom and a polar aprotic solvent, the resulting method provides robustness with a detection range of sulphate ion from a sub-nmol level to a nmol level. It has been shown that by using a methanol mixture with 3-metoxy-1-propanol, the sulphate ion detection can reach sub-µg (see Example 10), which is 10 times lower than the detection levels in the prior art. The method may measure sulphate ion as low as 17 ng.

In a second aspect of the present invention, there is provided a method of manufacturing a sulfohydrolase, comprising expressing a sulfohydrolase in a cell culture, and analyzing the enzymatic activity of the sulfohydrolase by colorimetric analysis.

In embodiments of the methods disclosed herein, the colorimetric analysis comprises use of a barium-rhodizonate complex in solution. The barium-rhodizonate complex may be defined as in Figure 1. The barium-rhodizonate complex in solution is at least partly solvated.

In some embodiments, an amount of a barium-rhodizonate complex in solution is measured in the presence of any sulphate ions that have been enzymatically removed from a substrate by said sulfohydrolase. The substrate contains at least one sulphur-heteroatom bond.

In some embodiments, the method of manufacturing a sulfohydrolase comprises a step wherein the enzymatic activity of the produced sulfohydrolase is colorimetrically measured according to the first method aspect as described herein. In some embodiments, the colorimetric analysis further comprises contacting the sulfohydrolase with the substrate, thereby allowing enzymatic removal of sulphate ions from said substrate(s).

A principle of the method is that the sulphate ions compete with the barium-rhodizonate complex for barium ions. Sulphate ions withdrawn from the substrate by the sulfatase replace rhodizonate in said barium-rhodizonate complex to form barium-sulphate (BaSO₄) and rhodizonate. In some embodiments, any sulphate ions being present form barium-sulphate with barium ions. This provides for a reduction of barium-rhodizonate complex in solution.

In the context of the present invention, "enzymatic activity" should be understood as the activity of an enzyme on a substrate, specifically, the ability of an enzyme to cleave a substrate.

Analysis of the activity of an enzyme provides a measure of the performance of said enzyme for said substrate. The measurement of performance may for instance be used for the testing of the following non-limiting parameters: potency, optimal pH and temperature, inhibitors and cofactors, stability and comparability, and kinetic parameters. In some embodiments, the step of colorimetric analysis comprises continuously measuring a reduction of barium-rhodizonate complex during enzymatic removal of said any sulphate ions from said substrate. Continuously could be understood as, for example, the repeated collection of data points of, for example, an absorbance, at different time points during a reaction. Such data points may be used for an absorbance vs time plot, useful for example in determination of kinetic parameters. As the method of analyzing the enzymatic activity of a sulfatase is a direct measurement of activity, solitary enzyme kinetics may be possible to conduct. By direct measurement is meant that the actual absorbance measured is due to activity of said enzyme, no item that converts the progress of the reaction into an observable quantity is required. In some embodiments, the step of colorimetric analysis comprises determining at least one kinetic parameter, such as a kinetic parameter selected from *K*_{cat} and *K*ₘ.

Colorimetric analysis may refer to measuring the absorbance at a wavelength in which a light-absorbing molecule, such as a color reagent, absorbs light. The measurement may be performed spectrophotometrically, by the use of a spectrophotometer.

In some embodiments, the amount of barium-rhodizonate is measured spectrophotometrically. In some embodiments, the method comprises measuring an absorbance of a barium-rhodizonate complex. The amount of barium-rhodizonate may be measured spectrophotometrically at a wavelength of 480-520 nm, such as 500-520 nm, such as 510 nm. The barium-rhodizonate complex is the item absorbing light at 480-520 nm. Barium-sulphate is not measured in said colorimetric analysis, as it comprises no absorbing portions.

In some embodiments, the colorimetric analysis comprises comparing the measured absorbance to a standard curve with known sulphate concentrations. The absorbance may be inversely proportional to the amount of any sulphate being enzymatically removed from the substrate. As understood by persons of skill in the art, the sulphate concentrations cover the amounts expected to be formed in the method when in use. The standard curve further comprises barium and rhodizonate at predetermined concentrations, relating to the concentrations in the method when in use.

In some embodiments, the ratio of said solution comprising methanol and at least one solvent selected from the group of an alcohol with 2-6 carbons, an ether alcohol with 4-6 carbons, a heterocycle with at least one heteroatom and a polar aprotic solvent may be present in a ratio of between 8:2 to 6:4 (v/v), such as 7:3 (v/v). That is, for example, taking 8 volumes methanol and 2 volumes of said further solvent, or taking 6 volumes methanol and 4 volumes of said further solvent or one may take 7 volumes methanol and 3 volumes of said further solvent. It is understood by persons of skill in the art that in the light of the example ratios above, the method encompasses taking any volumetric amount of methanol of between 6-8 volumes and further adding the corresponding volume or weight (if solid) of said further solvent for the final solution to add up to 10.

In the context of the present invention, "solvent" should be understood as a liquid or a solid, which is miscible or soluble in aqueous media.

In some embodiments, the barium-rhodizonate complex during said step of measuring is present in a solution having a pH at room temperature of from 3 to 5.5, such as from 4.1 to 4.8.

In some embodiments, the molar ratio between barium and rhodizonate is from 0.3 to 1.0, such as 0.7.

In some embodiments, the ratio (v/v) of solvent to methanol is from 0.1 to 1, preferably from 0.2 to 0.4.

In some embodiments, the solution comprises methanol and at least one solvent selected from a group of solvents. The at least one solvent may be an alcohol with 2-6 carbons, such as ethanol, propanol, 2-propanol, 2-butanol, tert-butanol, 1,3-propanediol, 1,4-butanediol, 1,3-butanediol, 1,5-pentanediol, 3-methyl-1,3-butanediol, 2-methyl-2,4-pentanediol or 1,6-hexanediol. In one embodiment, the at least one solvent is ethanol. In one embodiment, the at least one solvent is propanol. In one embodiment, the at least one solvent is 2-propanol. In one embodiment, the at least one solvent is 2-butanol. In one embodiment, the at least one solvent is tert-butanol. In one embodiment, the at least one solvent is 1,3-propanediol. In one embodiment, the at least one solvent is 1,4-butanediol. In one embodiment, the at least one solvent is 1,3-butanediol. In one embodiment, the at least one solvent is 1,5-pentanediol. In one embodiment, the at least one solvent is 3-methyl-1,3-butanediol. In one embodiment, the at least one solvent is 2-methyl-2,4-pentanediol. In one embodiment, the at least one solvent is 1,6-hexanediol.

The at least one solvent may be an ether alcohol with 4-6 carbons, such as 3-methoxy-1-propanol, 2-propoxyethanol, 2-butoxyethanol or 1-metoxy-2-propanol. In one embodiment, the at least one solvent is 3-methoxy-1-propanol. In one embodiment, the at least one solvent is 2-propoxyethanol. In one embodiment, the at least one solvent is 2-butoxyethanol. In one embodiment, the at least one solvent is 1-metoxy-2-propanol.

The at least one solvent may be a heterocycle with at least one heteroatom, such as tetrahydrofuran, furfuryl alcohol, 1,4-dioxane, N-methyl-2-pyrrolidone, pyridine, pyrazine, benzoic acid, benzylamine or caffeine. In one embodiment, the at least one solvent is tetrahydrofuran. In one embodiment, the at least one solvent is furfuryl alcohol. In one embodiment, the at least one solvent is 1,4-dioxane. In one embodiment, the at least one solvent is N-methyl-2-pyrrolidone. In one embodiment, the at least one solvent is pyridine. In one embodiment, the at least one solvent is pyrazine. In one embodiment, the at least one solvent is benzoic acid. In one embodiment, the at least one solvent is benzylamine. In one embodiment, the at least one solvent is caffeine.

The at least one solvent may be a polar aprotic solvent, such as acetone, acetonitrile, urea, dimethylformamide, dimethyl sulfoxide or dimethyl sulfone. In one embodiment, the at least one solvent is acetone. In one embodiment, the at least one solvent is acetonitrile. In one embodiment, the at least one solvent is urea. In one embodiment, the at least one solvent is dimethylformamide. In one embodiment, the at least one solvent is dimethyl sulfoxide. In one embodiment, the at least one solvent is dimethyl sulfone.

Selecting a least one solvent from any of the groups described above may allow for a solution in which a barium-rhodizonate complex is at least partly dissolved. Solvation of the complex may provide for a more accurate analysis of the enzymatic activity in the method described above.

The methods as disclosed herein involves colorimetric analysis of enzymatic activity. In some embodiments, the method comprises further steps.
For instance, the method may comprise a step of a): contacting the sulfohydrolase with the substrate, thereby allowing enzymatic removal of sulphate from the substrate and forming a mixture comprising sulfohydrolase, substrate and any sulphate being removed from the substrate. Furthermore, the method may comprise a step of b): providing a methanol solution comprising barium to the mixture. This may allow for formation of barium-sulphate from barium and the any sulphate, and may allow for formation of a mixture comprising sulfohydrolase, substrate, any barium-sulphate and remaining barium. Finally, the method may comprise a step of c): providing a solvent comprising rhodizonate to the mixture, thereby forming barium-rhodizonate with the remaining barium. The rhodizonate comprised in said solvent is preferably an alkali metal rhodizonate, such as lithium, sodium or potassium rhodizonate. Contacting said esterase and said substrate could be understood as bringing them in close proximity, by for example adding the two to a reaction mixture in for example a vial, whereby they may be brought into contact by diffusion. Enzymatic removal of sulphate is typically enzymatic hydrolysis of sulphonate esters. Furthermore, the sulphate removed by the enzyme is typically in ionic form, i.e. the sulphate is typically sulphate ion(s). The person of skill in the art will understand that the steps of a), b) and c) can be performed in any order, such as b), c), a); such as b), a), c); such as c), a), b); such as c), b), a); such as a), c), b); such as a), b), c). In some embodiments, step a) may be followed by an incubation of the mixture for a time period of at least 1 minute, such as at least 15 minutes, such as at least 20 minutes, such as for approximately 30 minutes, or for a time period of from 15 minutes to 24 hours, such as from 20 minutes to 60 minutes, such as from 20 minutes to 40 minutes. An optional incubation may be carried out at a temperature of approximately 0, 25, 30, 37, or 40 °C. Incubation time and/or temperature may be adapted to the specific sulfohydrolase, thereby allowing said enzymatic removal to take place.

In some embodiments, a curing step may be beneficial for establishing linearity in the method described. This may prevent precipitation of molecules from the mixture due to e.g. slow isomerization of rhodizonate in solution. A curing step may comprise incubation at room temperature under 800-1300 Lx (normal laboratory illumination on a bench) and further room temperature incubation in a dark room. A benefit of some embodiments of the present invention is that by using a mixture of methanol and at least one of 3-methoxy-1-propanol, 2-propoxyethanol and 2-butoxyethanol, different incubation treatments for the curing step of the rhodizonate solution were no longer necessary (see Example 7).

In some embodiments of, in particular, the manufacturing method aspect as disclosed herein, the sulfohydrolase is produced in cell culture. The sulfatase as described herein can be produced by recombinant techniques using eukaryotic, such as mammalian (including human), expression systems using conventional methods known to persons of skill in the art. Suitable cell lines for production of the sulfatase are known to persons of skill in the art, and examples include *Pichia pastoris, Saccharomyces cerevisiae*, algae, moss cells, plant cells such as carrot cells, and mammalian cells such as CHO, HEK-293, and HT1080. In particular embodiments wherein the sulfohydrolase is a modified sulfamidase, examples of suitable manufacturing methods are disclosed in Example 1 and in WO 2015/150490. In some embodiments, the sulfohydrolase is produced at a large scale.

A sulfohydrolase should be understood as an enzyme that catalyzes the removal of a covalent bonding sulphate portion (-SO₃⁻) from a substrate. Removal of such a group from a substrate is a result of the enzyme's activity on a sulphur bond. Non-limiting examples may be any enzyme acting on a glycosaminoglycan or any enzyme removing sulphate groups from sulphate derivatives. In some embodiments, the sulfohydrolase is selected from the group consisting of sulfamidase, iduronate 2-sulfatase, arylsulfatase A, arylsulfatase B, arylsulfatase E, arylsulfatase F, arylsulfatase G, arylsulfatase H, arylsulfatase I, arylsulfatase J, arylsulfatase K, N-acetylgalactosamine-4-sulfatase, N-acetylgalactosamine-6-sulfatase, N-acetylglucosamine-6-sulfatase, N-sulphoglucosamin sulphohydrolase, glucosamine 3-sulphate sulphatase, glucoronate 2-sulphate sulphatase, steryl-sulfatase, and extracellular sulfatase Sul-1.

In some embodiments, the sulfohydrolase is sulfamidase.

In some embodiments, the sulfamidase is a modified sulfamidase. A modified sulfamidase is understood by persons of skill in the art to be any modification to a native or recombinant sulfamidase, such as modifications in the gene sequence corresponding to said sulfamidase, such as posttranslational modifications, or such as chemical modifications. In particular, a modified sulfamidase may have an altered activity with respect to glycan recognition receptors. However, the enzymatic or catalytic specificity and/or activity is preferably not altered by said modification. Specific examples of modifications to a sulfamidase are disclosed in WO 2015/150490. For instance, a modified sulfamidase can be prepared by reacting a glycosylated sulfamidase with an alkali metal periodate for a time period of no more than 4 h, followed by reacting said sulfamidase with an alkali metal borohydride for a time period of no more than 2 h; thereby modifying glycan moieties of the sulfamidase and reducing the activity of the sulfamidase with respect to glycan recognition receptors. A modified sulfamidase resulting from such modification comprises substantially no epitopes for glycan recognition receptors. Thus, natural glycan moieties of said sulfamidase are disrupted; typically by single bond breaks and double bond breaks. In particular, the extent of single bond breaks per total bond breaks is at least 60 % in oligomannose glycans.

The methods as disclosed herein analyses the activity of a sulfohydrolase acting on a sulphur-heteroatom bond within a substrate. The substrate thus contains at least one sulphur-heteroatom bond (e.g., RO•SO₃⁻ or RN•SO₃⁻). In some embodiments, the substrate is selected from the group consisting of a natural substrate and a synthetic substrate.

In some embodiments, the substrate is selected from the non-limiting group of heparin, heparan sulphate and dermatan sulphate, or a functional equivalent thereof. Heparin and heparan sulphate are large linear polysaccharides consisting of uronic acid-D-glucosamine repeating disaccharide subunits. Differences in N-sulphate, O-sulphate and N-acetyl groups results in a large variation of complex sequences.

In some embodiments, a synthetic substrate may be preferred over a natural substrate. In such embodiments, the substrate is selected from the non-limiting group of non-reducing end saccharide-sulfonates (R•SO₃⁻), non-reducing end saccharide-sulfamates (RN•SO₃⁻), 4-methylumbelliferyl α-L-idopyranosiduronic acid 2-sulphate (MU-αldoAS), 4-methylumbelliferyl α-D-N-sulphoglucosaminide (MU-αGlcNS), 4-methylumbelliferyl β-D-galactopyranoside-6-sulphate (MU-βGal6S), sulfatide, cerebroside sulfate, oligosaccharides of heparin and heparan sulfate, fondaparinux fragments and heparin disaccharide I-S.

In some embodiments, the substrate is 4-methylumbelliferyl-α-D-N-sulphoglucosaminide (MU-αGlcNS).

Although the colorimetric measurement of sulphate ions as disclosed herein has primarily been tested within the field of determination of enzymatic activity of a sulfohydrolase, the general steps of the method are considered equally applicable in any other field where determination of an amount sulphate ions is desired. In the present methods, sulphate ions are generated by means of enzymatic cleavage. But as the skilled person will understand, sulphate ions can be generated by other means, and the method may be based on use of only barium, rhodizonate and sulphate in solution.

In a third aspect of the present invention, there is provided a method of analyzing an amount of sulphate ions in a sample, said method comprising a step of colorimetric analysis. Said colorimetric analysis comprises colorimetrically measuring an amount of a barium-rhodizonate complex in the presence of any sulphate ions in said sample, said amount of barium-rhodizonate complex being inversely proportional to the amount of sulphate ions in said sample, wherein said barium-rhodizonate complex during said step of measuring is present in a solution comprising methanol and at least one solvent selected from the group of an alcohol with 2-6 carbons, an ether alcohol with 4-6 carbons, a heterocycle with at least one heteroatom and a polar aprotic solvent.

An analysis such as the one described in the above third aspect may enable measurement of any sulphate in a sample which may or may not contain free sulphate ions. The analysis may be qualitative, with the aim of determining any presence of sulphate, or quantitative, with the aim of determining the amount of any sulphate. In particular, any sulphate ions present in the sample form barium-sulphate with barium ions, whereby a reduction of barium-rhodizonate complex in solution is provided. The amount of barium-rhodizonate may be measured spectrophotometrically, optionally as set out in connection with the first aspect above.

Several fields of application for a method according to the third aspect are contemplated. For example, said method could be useful for analyzing and/or measuring any residual of free sulphate for compounds or materials. In addition, said method could be useful in food industry, for analyzing the presence of any sulphate ions in e.g. a beverage. Moreover, said method could be useful for analyzing sulphate released during pyrolysis of a compound or material, and/or to analyze fee sulphate of particulate matters in air.

In one embodiment of the third aspect, said sample is an organic sample. In another embodiment, said sample is an inorganic sample.

In one embodiment of the third aspect, said sample is an aqueous sample, such as a sample selected from water, a beverage, a biological fluid, a growth media and sewage.

In one embodiment of the third aspect, said sample is filtered from air. This could e.g. enable analysis of any water-soluble sulphate filtered from the air.

Whereas aqueous samples may be analyzed directly, airborne sample may be collected in an appropriate manner, such as on an appropriate filter followed by elution of sulphate in e.g. water.

In a fourth aspect of the present invention, there is provided use of a barium-rhodizonate complex in solution to determine enzymatic activity of a sulfohydrolase, said complex being formed by combination of a methanol solution comprising barium with a solution comprising alkali metal rhodizonate in at least one solvent selected from the group of an alcohol with 2-6 carbons, an ether alcohol with 4-6 carbons, a heterocycle with at least one heteroatom and a polar aprotic solvent. Specific example solvents are described in the itemized list of embodiments and elsewhere herein. Said methanol solution comprising barium is referred to as a "first solution" whereas said solution comprising alkali metal rhodizonate comprising at least one solvent is referred to as a "second solution" herein.

In some embodiments, there is provided use of a barium-rhodizonate complex in solution to determine enzymatic activity of a sulfatase.

In some embodiments, there is provided use of a barium-rhodizonate complex in solution to determine enzymatic activity of a modified sulfatase.

Said combination of a first and second solution is in one embodiment carried out in the presence of any sulphate ions that have been enzymatically removed from a substrate by said sulfohydrolase. As defined in other aspects herein, said substrate may contain at least one sulphur-heteroatom bond. The amount of barium-rhodizonate complex being formed in solution is inversely proportional to the enzymatic activity of said sulfohydrolase.

It should be understood that the embodiments disclosed in connection with the method aspects are equally relevant, if applicable, to the use aspect as disclosed herein. In particular, examples of solvents and suitable ranges of the same are disclosed elsewhere herein.

The invention will be further illustrated by the following non-limiting drawings and examples.

### Brief description of the drawings

Figure 1 is a drawing depicting solvent effects on barium-rhodizonate assays according to the prior art (Fig. 1A) and to an embodiment of the present invention (Fig. 1B). Rhodizonate, represented by C₆O₆²⁻ in both A and B, is a symmetric/aromatic dianion which causes an orange-red shade when coordinated with Ba²⁺. Coordination with Ba²⁺ causes an absorption shift of the chromophore from 480 nm to 510 nm. The reaction is irreversible because the end product of BaSO₄ has the lowest *Kₛₚ.* In Fig. 1A, there is a risk of precipitation of the reactant [C₆O₆²⁻]Ba²⁺ because of a poor solvation. Precipitation might interrupt the reaction process, as indicated by dashed arrows. In Fig. 1B, depicting an exemplary assay of the present invention, the reactant (C₆O₆²⁻) in transition is well-solvated, and the reaction takes place stoichiometrically to the end, as indicated by the solid arrows.

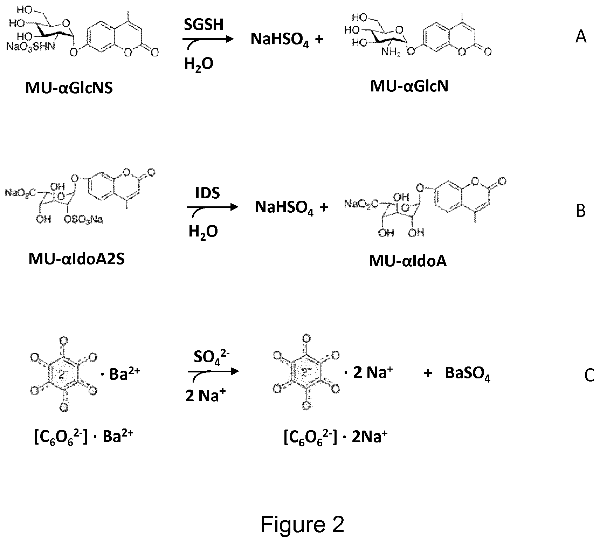

Figure 2 is a drawing showing reaction schemes of exemplary enzyme activity assays in accordance with some embodiments. In Figure 2A, sulfamidase (SGSH) acts on a sulphur-heteroatom bond of a synthetic substrate (MU-αGlcNS), thereby producing free inorganic sulphate. In Figure 2B, iduronate-2-sulfatase acts on a sulphur-heteroatom bond of a synthetic substrate (MU-αldoAS), thereby producing free inorganic sulphate. Figure 2C depicts free sulphate, as formed in Fig. 2A or B, competing with the chromophore of the barium-rhodizonate complex ([C₆O₆²⁻]•Ba²⁺, *Kₛₚ* (25°C) = 6 x 10⁻⁹), forming BaSO₄ (*Kₛₚ* (25°C) = 1 x 10⁻¹⁰). The formed rhodizonate sodium salt lacks color. The differential solubility draws the reaction to the end. All free sulphate product formed in Fig. 2A and B can be stoichiometrically determined by the barium-rhodizonate reaction of Fig. 2C.

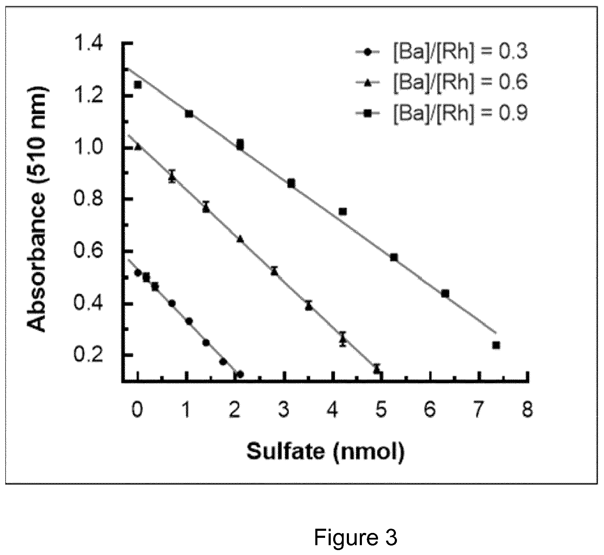

Figure 3 shows a graph in which different molar ratios of barium to rhodizonate are plotted for absorbance vs concentration of sulphate (ion), in accordance with some embodiments. Molar ratios ([Ba]/[Rh)]) are 0.3 (cycle), 0.6 (triangle) and 0.9 (square), whose sulphate calibration curves were made by 175 pmol to 2125 pmol, 0.7 nmol increment to 4.9 nmol, and 1.05 nmol increment to 7,35 nmol respectively.

### Examples

### Reference Example 1: Cultivation, purification, characterization and modification of Sulfamidase

### Construction of expression vectors for sulfamidase

Synthetic genes encoding human sulfamidase were synthesized by Geneart (Life Technologies), both in codon optimized versions for *H. sapiens* or C. *griseus* (CHO cells) and the original human sequence. The synthetic genes were cloned in different mammalian expression vectors, such as pcDNA3.1 (+) (Invitrogen) orpQMCF1 (Icosagen).

### Production of sulfamidase

Two transient expression systems were evaluated for sulfamidase production, transient expression in HEK293 cells using pcDNA3.1 (+) vectors and the Quattromed Cell Factory (QMCF) episomal expression system (Icosagen AS) using the pQMCF1 vector. In both systems cells were grown in standard medium and secreted protein was harvested typically 6-8 days after transfection. In addition, a stable cell line established using a commercially available CHO expression system was evaluated for production of sulfamidase. Sulfamidase was captured from medium by anion exchange chromatography (AIEX) on a Q sepharose column (GE Healthcare) equilibrated with 20 mM Tris, 1 mM EDTA, pH 8.0 and eluted by a NaCl gradient. Captured sulfamidase was further purified by 4-Mercapto-EthylPyridine (MEP) chromatography; sulfamidase containing fractions were loaded on a MEP HyperCel chromatography column and subsequently eluted by isocratic elution in 50 mM NaAc, 0.1 M NaCl, 1 mM EDTA, 1 mM OTT, pH 4.6. Final polishing was achieved by cation exchange chromatography (CIEX) on a SP Sepharose FF (GE Healthcare) column equilibrated in 25 mM NaAc, 2 mM OTT, pH 4.5. A NaCl gradient was used for elution. Purity and identity of sulfamidase batches from the different expression systems were analyzed by SDS-PAGE and MALDI-TOF-MS, data not shown.

### Chemical modification of sulfamidase

Sulfamidase produced as set out above was chemically modified in accordance with the alternative modifying methods 1 to 5 set out below.

Modifying method 1: Recombinant sulfamidase was oxidized by incubation with 20 mM sodium meta-periodate at 0 degrees centigrade in the dark for 120 min in phosphate buffers having a pH of 6.0. Glycan oxidation was quenched by addition of ethylene glycol to a final concentration of 192 mM. Quenching was allowed to proceed for 15 min at 6 degrees centigrade before sodium borohydride was added to the reaction mixture to a final concentration of 50 mM. After incubation at 0 degrees centigrade for 120 min in the dark, the resulting sulfamidase preparation was ultrafiltrated against 20 mM sodium phosphate, 100 mM NaCl, pH 6.0.

Modifying method 2: Performed as modifying method 1 with the exception that the concentration of sodium borohydride in the reduction step was 10 mM.

Modifying method 3: Recombinant sulfamidase was oxidized by incubation with 10 mM sodium *meta*-periodate at 0 degrees centigrade in the dark for 180 min in acetate buffer having an initial pH of between 4.5 to 5.7. Glycan oxidation was quenched by addition of ethylene glycol to a final concentration of 192 mM. Quenching was allowed to proceed for 15 min at 6 degrees centigrade before sodium borohydride was added to the reaction mixture to a final concentration of 25 mM. After incubation at 0 degrees centigrade for 60 min in the dark, the resulting sulfamidase preparation was ultrafiltrated against 10 mM sodium phosphate, 100 mM NaCl, pH 7.4.

Modifying method 4: Recombinant sulfamidase was oxidized by incubation with 10 mM sodium *meta*-periodate at 8 degrees centigrade in the dark for 60 min in acetate buffer having an initial pH of 4.5. Glycan oxidation was quenched by addition of ethylene glycol to a final concentration of 192 mM. Quenching was allowed to proceed for 15 min at 6 degrees centigrade before sodium borohydride was added to the reaction mixture to a final concentration of 25 mM. After incubation at 0 degrees centigrade for 60 min in the dark, the resulting sulfamidase preparation was ultrafiltrated against 10 mM sodium phosphate, 100 mM NaCl, pH 7.4.

Modifying method 5: Recombinant sulfamidase was oxidized by incubation with 10 mM sodium *meta*-periodate at 8 degrees centigrade in the dark for 60 min in acetate buffer having an initial pH of 4.5. Glycan oxidation was quenched by addition of ethylene glycol to a final concentration of 192 mM. Quenching was allowed to proceed for 15 min at 6 degrees centigrade before sodium borohydride was added to the reaction mixture to a final concentration of 25 mM. After incubation at 0 degrees centigrade for 45 min in the dark and quenching the reaction with 0.1 M acetone, the resulting sulfamidase preparation was ultrafiltrated against 10 mM sodium phosphate, 100 mM NaCl, pH 7.4.

### Example 2: Barium rhodizonate activity assay

A reaction mixture comprising solutions of 0-7 nmol sulphate, 7 µL sulfamidase (Example 1) and 7 µL substrate (MU-αGlcNS) was incubated at pH 6.5 in a microtiter plate for 30-40 min at 37 °C, thereafter 120 µL barium in methanol and 60 µL rhodizonate solution was added. The plate was covered with a black lid, and incubated for 25 min (room temperature, gentle sharking). Absorbance was measured spectrophotometrically at 510 nm. The assay was used in Examples 3-5. Reaction schemes of exemplary assays are depicted in Figure 2.

### Example 3: Temperature & pH optima for the enzyme in the assay

Activity was measured as described in Example 2, but at a varying temperature and a different pH in order to find the conditions for optimum activity for sulfamidase and modified sulfamidase of Example 1.

For profiling of a pH optimum curve, the enzyme activity reaction was fixed at 37 °C for 40 min. For test of thermo-sensitivity of the enzyme activity, the enzyme activity was tested at pH 6.5 under the described conditions except varying a temperature for the enzyme reaction and using tight screw tubes instead of a sealing incubation plate for the enzyme incubation above 37 °C to prevent evaporating.

Test enzyme- and substrate dilution buffers were prepared with varying pH values at; 3.5, 5.0, 6.5, 8.0, and 9.5 respectively. The two purified enzyme samples, unmodified sulfamidase or sulfamidase (15-16 µg), modified in accordance with a method as disclosed in WO 2015/150490, were set to the pH values defined above prior to the assay.

All calculated activity data were plotted in software GraphPad Prism and further analyzed in the software MODDE (version 12) with two quantitative factors: pH (3.5-9.5) at 5 levels, temperature (25-95 °C) at 10 levels and two regular responses: activity (U/mg) for modified sulfamidase and unmodified sulfamidase (data not shown).

### Results

Both the unmodified and the modified sulfamidase had an optimum pH at 6 for activity. Overall, the unmodified and the modified sulfamidases seemingly had similar thermo-tolerance properties when compared in MODDE. The temperature optimum was 70 °C for the modified sulfamidase and 85 °C for the unmodified sulfamidase.

### Example 4: Enzyme kinetics

The enzymatic assay was performed under the conditions as described in Example 2. However, in this experiment the substrate MU-αGlcNS was diluted to 8 different concentrations; 50, 25, 20, 15, 10, 5, 2.50 and 1.25 mM before added in triplicates to start the reaction. The activity data were analyzed in the software GraphPad Prism using nonlinear regression for substrate (mM) vs. velocity (U/mg) to fit the Michaelis-Menten equation. The colorimetric analysis thus proved itself useful in determining kinetic parameters such as *K*_{cat} and *K*ₘ.

### Example 5: Assay procedure for solvent screening assay

Activity was measured as described in Example 2. The solvent screening assay was carried out for sulphate calibration series from 7 to 0 (1 nmol decrements). A reaction mixture of 0.2 mL comprising 13.6 nmol rhodizonate, 8 nmol Ba²⁺, 58% MeOH, 25% test solvent and 17% buffer solution (pH 4.8 at 25°C) in a covered round bottom plate, was incubated at room temperature on a plate shaker with gentle shaking for developing a color for 30 min. Thereafter, 155 µL of the incubated solution was transferred to a detection plate (half area flat bottom) and covered by a black lid before reading.

Absorbance was read spectrophotometrically at 510 nm, typically after 45 min of the color development, with subsequent tracking for 2-3 h to judge dyeing stability.

### Example 6: Identification of feasible solvents

In search of a water miscible solvent that, alone or mixed with methanol in an aqueous solution containing barium, absorbs light at around 510 nm, and from which rhodizonate does not precipitate, 46 different solvents (water-miscible liquids or solids soluble in aqueous media) were investigated.

The tested solvents were methanol, ethanol, propanol, 2-propanol, 2-butanol, tert-butanol, 1,5-pentanediol, 1,4-butanediol, 1,3-butanediol, 1,3-propanediol, 1,6-hexanediol, 2-methyl-2,4-pentanediol, 3-methyl-1,3-butanediol, ethylene glycol, 1,2-propanediol, glycerol, diethylene glycol, triethylene glycol, 3-methoxy-1-propanol, 2-butoxyethanol, 2-propoxyethanol, 1-methoxy-2-propanol, dimethoxyethane, furfuryl alcohol, tetrahydrofuran, 1,4-dioxane, N-methyl-2-pyrrolidone, pyridine, pyrazine, benzoic acid, benzylamine, caffeine, imidazole, 1-methylimidazole, pyrimidine, pyridazine, acetone, acetonitrile, urea, hexafluroisopropanol, dimethylformamide, dimethyl sulfoxide, dimethyl sulfone, guanidine (hydrochloride salt), methyl diethanolamine and ethylenediaminetetraacetic acid.

### Results

In the group of monohydric alcohols, methanol, ethanol, propanol, 2-propanol, 2-butanol and tert-butanol were identified as feasible solvents, for which color appeared almost instantly and was fully developed within 20 to 40 minutes. A "slow" and a "fast" color development type of useful solvents was identified. Typically, shorter carbon chain number solvents were faster, but methanol deviated from this pattern (30 min).

Methanol was identified as the sole solvent that could be used as single solvent in the assay without a risk of precipitating. It was also verified that a primary alcohol is better than a secondary one with a same carbon number in terms of precipitate risk and calibration linearity.

In the group of polyhydric alcohols, the 7 diols 1,5-pentanediol, 1,4-butanediol, 1,3-butanediol, propanediol, 1,6-hexanediol, 3-methyl-1,3-butanediol and 2-methyl-2,4-pentanediol were identified as feasible solvents, while the five diols ethylene glycol, 1,2-propanediol, glycerol, diethylene glycol and triethylene glycol were not.

It was found that vicinal diols and their ethers prevent rhodozinate from dyeing. Furthermore, the shorter the diol carbon chain, the longer in the color development (except for 1,3-propanediol). Interestingly, diols stabilize the color remarkably (over half day or even overnight), but their higher viscosity could give rise to transferring and mixing errors.

In the group of ether alcohols and dimethoxyethane, 3-methoxy-1-propanol, 2-butoxyethanol, 2-propoxyethanol, and 1-methoxy-2-propanol were identified as feasible solvents, while dimethoxyethane was possibly feasible.
Most of these hybrid alcohols showed an excellent impact on the method in terms of linearity, stability and usability. Compared with diols of same chain length, viscosity was largely decreased. The dyeing rate was slower compared to their precursor alcohols but faster compared to the diols having same carbon number. Extensive investigation verified that a secondary hydroxyl increased the risk of the precipitation of the dye during the assay.

In the group of pentacvclic, hexacvclic and aromatic cycles, furfuryl alcohol, tetrahydrofuran, 1,4-dioxane, N-methyl-2-pyrrolidone, pyridine, pyrazine, benzoic acid, benzylamine, and caffeine were identified as feasible solvents, while imidazole, 1-methylimidazole, pyrimidine, and pyridazine were not. Notably in the group, heterocyclic rings, aromatic compounds or N-methyl-2-pyrrolidone did not disturb the rhodizonate dyeing, nor were rings lacking a hydroxyl group.

In the group of acetone, acetonitrile, dimethyl sulfoxide and miscellaneous, acetone, acetonitrile, urea, hexafluroisopropanol, dimethylformamide, dimethyl sulfoxide, and dimethyl sulfone were identified as feasible solvents, while guanidine, methyl diethanolamine, and ethylenediaminetetraacetic acid were not.
Acetone and acetonitrile showed a very similar feature as ethanol, tetrahydrofuran and 1,4-dioxane in terms of fast dyeing and curvilinear patterns.

### Discussion

Solvents that gave rise to a linear dependency of the absorbance of rhodizonate vs the amount of sulphate were considered to be feasible. Conclusions based on structure and functional groups of the solvents could be drawn. The non-polar, polar protic and polar aprotic solvents and soluble ligands have captured a broad structural spectrum, which might fall into three categories based on their roles: 1) a color-accelerator (e.g., ethanol, 1-propanol and acetone), 2) a curvature-depressor (e.g., 3-methoxy-1-propanol, 2-methyl-2,4-pentanediol, and N-methyl-2-pyrrolidone together with a color-accelerator), and 3) a color-stabilizer (e.g., methanol, 1,5-pentanediol and 1,6-hexanedol).

It can also be concluded that strong chelates, vicinal diols and their ethers or amines, imidazole or pyrimidine or pyridazine rings interrupt the dye. Furthermore, a secondary hydroxyl increases a risk of the dye solubility, and hydrotropes that alone or together with others can associate the aromatic pi stacking spices (C₆O₆²⁻) may have the rectilinear effect.
The solvent effects on the reaction may also lie in the fact that rhodizonic acid is a weak diprotic acid whose ionization constants are unusually close (p*K₁* = 4.4; p*K₂* = 4.6) and whose barium salt has a poor solubility in water (*Kₛₚ* = 6 × 10⁻⁹ at 25°C).

### Example 7: Performance of selected solvents

In the following examples, the dyeing performance of a rhodizonate solution was tested. The rhodizonate solution was made using an organic solvent at 80% (v/v) in water comprising disodium rhodizonate at 227 µM and ascorbic acid at 5.7 mM. Different treatments to the solution were investigated for 8 selected solvents that were methanol, ethanol, propanol, 1,5-pentanediol, 3-methoxy-1-propanol, 2-butoxyethanol, 2-propoxyethanol and 1-methoxy-2-propanol. The dyeing reaction was started when the following 3 solutions were mixed: 1) 60 µL of the rhodizonate solution, 2)120 µL of the barium solution (67 µM BaCl, 533 µM NaHCO₃, 67 mM acetic acid, 93% methanol and 17% water, pH 4.8 at 25°C), and 3) 14 µL of calibration series containing sulfate from 0 to 4.9 nmol in 0.7 nmol increment in water. The dyeing incubation and the test procedures were as described in *Example 6.*

Evaluations focused on precipitate, dyeing durability (45 -135 min), curve linearity (a normal r² of 0.90-0.99), precision (data scattering) and sensitivity (line slope).

### Light exposure

Light exposure of the solutions comprising disodium rhodizonate under 800 Lx (a normal laboratory illumination on the bench) more than 30 min deteriorated the linearity, and particularly resulted in precipitating for the rhodozinate comprising solutions of ethanol, propanol and 1-methoxy-2-propanol. However, a short exposure under 800-1300 Lx for 15 min in combination of 2-3 h stay in the dark could improve linearity and precision, and prevented precipitating.

A short light exposure followed by 0.5 h stay in dark at room temperature promoted linearity and stability of the rhodizonate solutions. Thereafter, for some solvents, such as 1-metoxy-2-propanol, 2-butoxyethanol and 1,5-pentanediol, the quality of the rhodizonate solutions declined gradually after a prolonged stay in dark at room temperature from 0.5 h to 2 h.

Without a light exposure of the rhodizonate solution, using the solvents of methanol, ethanol and propanol, the linear calibration series could result in a pronounced polynomial curvature against the calibration sulfate series.

### Freezing

Surprisingly, a single freezing process (< -70°C, overnight) enhanced or restored the line slope and dyeing stability, alleviated the curvature, and relieved the risk of precipitate for many of the solvents comprising disodium rhodizonate. Moreover, using 3-methoxy-1-propanol, 2-butoxyethanol and 2-propoxyethanol to prepare the disodium rhodizonate solution, made treatments of light exposure, short room stay, and freezing unnecessary.

### Discussion

This experiment confers that disodium rhodizonate in solution is not stable under light in room temperature for more than 30 minutes, by showing precipitation, poor linearity or polynomial curvature (rhodizonate absorbance vs sulphate concentration) in the assay. However, a treatment of 15 minutes of exposure to light with subsequent dark room exposure for 2-3 h established linearity and stability for a majority of the selected solvents. Surprisingly, deep freezing resulted in the most positive effect for rhodizonate reagents over the light exposure and the room incubation. The rhodizonate reagent benefitted from being stored in the freezer. More beneficially, by using 3-methoxy-1-propanol, 2-butoxyethanol and 2-propoxyethanol, to prepare the disodium rhodizonate solution, freezing and other treatments became unnecessary. What is most intriguing is that the polynomial curvature feature was seen by methanol, whose rhodizonate solution was pronounced curvature for the calibration sulfate series without a light exposure, but became a perfectly linear after a short light exposure or freezing (data not shown). This suggests that the curvature was not resulted from insolubility mechanism since methanol alone had never resulted in a rhodizonate precipitate. Thus, the polynomial curve may be due to the hydration mechanism (see Figure 1).

### Precipitation

Surprisingly, precipitating was only avoided by using methanol in the assay. Methanol alone never caused precipitating. Thus, a ratio (v/v) of solvent comprising rhodizonate to methanol is feasibly from 0 to 1, preferably 0.2 ∼ 0.4 (data not shown).

### Dyeing performance of mixed solvents

The rhodizonate comprising solvents ethanol, propanol, 3-methoxy-1-propanol, 2-propoxyethanol, 2-butoxyethanol and 1-metoxy-2-propanol were mixed with barium in methanol and tested for their dyeing performances.

To investigate the impact of freezing on dye potency, samples were treated with a light exposure under 1300 Lx for 15 min, with a subsequent incubation in dark for 0.5 h or 2 h (all in room temperature). Thereafter, samples were either tested directly (fresh) or stored in -70 °C overnight before thawed and tested (frozen).

Table 1 highlights the key observations of the tests, which tell the dyeing performance of the individual solvents comprising disodium rhodizonate in mixing with barium in methanol.

**Table 1. Evaluation data of six solvents comprising rhodizonate for dyeing performance with methanol comprising barium. The solvents used were ethanol (EtOH), propanol (PrOH), 3-metoxy-1-propanol (13M1P), 2-propoxyethanol (2BE), 2-butoxyethanol (17) and 1-metoxy-2-propanol (1M2P)**

| **Solvent** | | **EtOH** | | **PrOH** | | **3M1P** | | **2PE** | | **2BE** | | **1M2P** | |
|---|---|---|---|---|---|---|---|---|---|---|---|---|---|
| Test | (h)^{d} | Fresh | Froz | Fresh | Froz | Fresh | Froz | Fresh | Froz | Fresh | Froz | Fresh | Froz |
| Coloring^{a} | 0.5 | Fast | Fast | Fast | Fast | Slow | Slow | Slow | Slow | Slow | Slow | Slow | Slow |
| | 2.0 | Fast | Fast | Fast | Fast | Slow | Slow | Slow | Slow | Slow | Slow | Slow | Slow |
| Precipitating^{b} | 0.5 | -/+ | - | -/+ | - | - | - | - | - | - | - | -/+ | - |
| | 2.0 | + | - | + | - | - | - | - | - | - | - | -/+ | - |
| Curvature^{c} | 0.5 | + | + | + | + | - | - | - | - | - | - | - | - |
| | 2.0 | + | + | + | + | - | - | - | - | - | - | - | - |

| | | | | | | | | | | | | | |
|---|---|---|---|---|---|---|---|---|---|---|---|---|---|
| a. The dyeing color was fully developed after 0.5 (fast) or by 1 h (slow) b. The symbols indicate precipitate never being seen (-), being visible at times over 0.5 (-/+) and being visible within 0.5 h (+) c. The symbols indicate Excel XLfit model 151 a polynomial curvature line (+) and model 100 a straight line (-). d. The rhodizonate solution was exposed to light 1300 Lx for 15 min and then incubated at room temperature for 0.5 h or 2.0 h respectively. Thereafter, the solution was tested without freezing (fresh) or tested after freezing overnight (froz = frozen). | | | | | | | | | | | | | |

The impact on the dye quality (e.g., line slope) by freezing was tested. In the frozen test occasions, the color stability was also tracked in each 15-min interval from 45 to 150 min, which confirmed the somewhat curvature feature of ethanol or propanol (data not shown).

### Discussion

These results confirmed all the previous observations regarding light exposure, room incubation and freezing (see above), as well as the risk of precipitate resulted from the secondary -OH group on 1-metoxy-2-propanol. The ether alcohol group of 3-methoxy-1-propanol, 2-propoxyethanol and 2-butoxyethanol were all slow coloring but identified to be the best regarding linearity, stability, and sensitivity in the assay.

### Example 8. Testing NMP, DMF and DMSO

The three polar aprotic solvents, N-methyl-2-pyrrolidone, dimethyl formamide and dimethyl sulfoxide were further studied because they are frequently used as the solvents for reactions involving salts and nucleophilic substitutions. With a mixture of this aprotic solvent to methanol (1:2 v/v), the dye developed slowly and took 1.5 h to be completed. However, when mixed with a third solvent such as acetone, acetonitrile, tetrahydrofuran, 1,4-dioxane and 1,6-hexanediol, the dye developed by 0.5 h and the linearity against sulphate became ideal. Extensive tests were focused on N-methyl-2-pyrrolidone and dimethyl sulfoxide and the data showed the percentage of them in the dyeing solution plays an important role (data not shown). Ideal dyeing media in terms of the linearity (r² > 0.99), the dyeing speed (< 45 min) and the color stability (45 - 135 min), were obtained as follows:

| | |
|---|---|
| 10 ± 3 % (v/v) | N-methyl-2-pyrrolidone or dimethyl sulfoxide |
| 15 ± 3 % (v/v) | Acetone, acetonitrile, ethanol or 1,4-dioxane |
| 57 ± 5 % (v/v) | Methanol |
| 18 ± 5 % (v/v) | Water |
| 12 ± 3 % (w/v) | 1,6-Hexanediol |

### Example 9: The pH range of the assay

The two solvent mixtures methanol/ethanol and methanol/3-methoxy-1-propanol were tested for preferred pH range. Pyridine instead of sodium carbonate was used to make a pH determined buffer series with pH from 3.2 to 5.9 at intervals of 0.2-0.7 in the dyeing reaction, since sodium ion over 8.3 mM might cause precipitate with the dye. Pyridine at the test concentrations did not interfere the dye function. To conclude, the dyeing worked in a weak acid range with pH values between 3 and 5.5 at room temperature, preferably at a pH value of around from 4.1 to 4.8.

### Example 10. The molar ratios of barium and rhodizonate

The effect of solvents methanol, ethanol, propanol, 3-methoxy-1-propanol, 2-propoxyethanol and 2-butoxyethanol on the varying ratios (mol/mol) of barium to rhodizonate was investigated. The results are depicted in Figure 3. The ratios were 0.3 (circle line) to cover 0-2.1 nmol sulphate being of a low limit 175 pmol (ca 17 ng), 0.6 (triangle line) to cover 4.9 nmol sulphate and 0.9 (square line) to cover 7.35 nmol sulphate respectively. Linear regression fitted independent duplicate data (r² > 0.99) with SD values (error bar), whose absorbance data were read out at 1 h after dyeing. The solvent was 58% methanol, 24% 3-methyoxy-1-propanol and 18% water by v/v.

### Discussion

Varying the molar ratio of barium to rhodizonate had an impact on the test range of sulphate. The barium/rhodizonate ratio improved the detection range of sulphate ion from a sub-nmol level to nmol levels, when assayed, for instance, with the mixed solvent of methanol and 3-methoxy-1-propanol. Furthermore, light exposure and/or short room stay before freezing did not influence the results, concluding that those pretreatments were not necessary.

### Itemized list of embodiments

1. A method of analyzing the enzymatic activity of a sulfohydrolase, comprising a step of colorimetric analysis, said colorimetric analysis comprising:
   colorimetrically measuring the amount of a barium-rhodizonate complex in the presence of any sulphate ions that have been enzymatically removed from a substrate by said sulfohydrolase, said substrate containing at least one sulphur-heteroatom bond and said amount of barium-rhodizonate complex being inversely proportional to the enzymatic activity of said sulfohydrolase,
   wherein said barium-rhodizonate complex during said step of measuring is present in a solution comprising methanol and at least one solvent selected from the group of an alcohol with 2-6 carbons, an ether alcohol with 4-6 carbons, a heterocycle with at least one heteroatom and a polar aprotic solvent.
2. A method of manufacturing a sulfohydrolase, comprising
   expressing a sulfohydrolase in a cell culture, and
   analyzing the enzymatic activity of said sulfohydrolase by colorimetric analysis.
3. The method of manufacturing a sulfohydrolase according to item 2, wherein said colorimetric analysis comprises use of a barium-rhodizonate complex in solution.
4. The method of manufacturing a sulfohydrolase according to item 3, wherein said use comprises measuring an amount of a barium-rhodizonate complex in solution in the presence of any sulphate ions that have been enzymatically removed from a substrate by said sulfohydrolase, said substrate containing at least one sulphur-heteroatom bond.
5. The method of manufacturing a sulfohydrolase according to any one of items 2 to 4, wherein the enzymatic activity of said produced sulfohydrolase is colometrically measured according to the method of item 1.
6. The method according to any one of the preceding items, wherein said colorimetric analysis further comprises contacting said sulfohydrolase with said substrate, thereby allowing enzymatic removal of sulphate ions from said substrate.
7. The method according to any one of the preceding items, wherein said any sulphate ions forms barium-sulphate with barium, whereby a reduction of barium-rhodizinate complex in solution is provided.
8. The method according to any one of the preceding items, wherein said step of colorimetric analysis comprises continuously measuring a reduction of barium-rhodizonate complex during enzymatic removal of said any sulphate ions from said substrate.
9. The method according to item 1, wherein said step of colorimetric analysis comprises determining at least one kinetic parameter, such as a kinetic parameter selected from *K*_{cat} and *K*ₘ.
10. The method according to any one of the preceding items wherein the amount of barium-rhodizonate is measured spectrophotometrically.
11. The method according to item 10, wherein the amount of barium-rhodizonate is measured spectrophotometrically at a wavelength of 480-520 nm, such as 500-520 nm, such as 510 nm.
12. The method according to item 10 or 11, wherein the measured absorbance is compared to a standard curve with known sulphate concentrations, the absorbance being inversely proportional to the amount of any sulphate being enzymatically removed from said substrate.
13. The method according to any one of the preceding items, wherein methanol and said at least one solvent is present in said solution in a ratio of between 8:2 to 6:4 (v/v), such as 7:3 (v/v).
14. The method according to any one of the preceding items, wherein said barium-rhodizonate complex during said step of measuring is present in a solution having a pH at room temperature of from 3 to 5.5, such as from 4.1 to 4.8.
15. The method according to any one of the preceding items, wherein the molar ratio between barium and rhodizonate is from 0.3 to 1.0, such as 0.7.
16. The method according to any one of the preceding items, wherein the ratio (v/v) of solvent to methanol is from 0.1 to 1, preferably from 0.2 to 0.4.
17. The method according to any one of the preceding items, wherein the alcohol with 2-6 carbons is selected from ethanol, propanol, 2-propanol, 2-butanol, *tert*-butanol, 1,3-propanediol, 1,4-butanediol, 1,3-butanediol, 1,5-pentanediol, 3-methyl-1,3-butanediol, 2-methyl-2,4-pentanediol, 1,6-hexanediol.
18. The method according to any one of the preceding items, wherein the ether alcohol with 4-6 carbons is selected from 3-methoxy-1-propanol, 2-propoxyethanol, 2-butoxyethanol and 1-methoxy-2-propanol.
19. The method according to any one of the preceding items, wherein the heterocycle with at least one heteroatom is selected from tetrahydrofuran, furfuryl alcohol, 1,4-dioxane, N-methyl-2-pyrrolidone, pyridine, pyrazine, benzoic acid, benzylamine and caffeine.
20. The method according to any one of the preceding items, wherein the polar aprotic solvent is selected from acetone, acetonitrile, urea, dimethylformamide, dimethyl sulfoxide and dimethyl sulfone.
21. The method according to any one of the preceding items, further comprising the steps of:
   a) contacting said sulfohydrolase with said substrate, thereby allowing enzymatic removal of sulphate from said substrate and forming a mixture comprising sulfohydrolase, substrate and any sulphate being removed from said substrate;
   b) providing a methanol solution comprising barium to said mixture of a), thereby allowing formation of barium-sulphate from barium and said any sulphate, and forming a mixture comprising sulfohydrolase, substrate, any barium-sulphate and remaining barium, and
   c) providing a solvent comprising alkali metal rhodizonate, such as lithium, sodium or potassium rhodizonate, to the mixture of b), thereby forming barium-rhodizonate with said remaining barium.
22. The method according to item 21, wherein contacting of a) is followed by an incubation of said mixture for a time period of from 1 minute to 24 hours, optionally at 37 °C.
23. The method according to any one of the preceding items, wherein said sulfohydrolase is produced in cell culture.
24. The method according to any one of the preceding items, wherein said sulfohydrolase is selected from the group consisting of sulfamidase, iduronate 2-sulfatase, arylsulfatase A, arylsulfatase B, arylsulfatase E, arylsulfatase F, arylsulfatase G, arylsulfatase H, arylsulfatase I, arylsulfatase J, arylsulfatase K, N-acetylgalactosamine-4-sulfatase, N-acetylgalactosamine-6-sulfatase, N-acetylglucosamine-6-sulfatase, N-sulphoglucosamin sulphohydrolase, glucosamine 3-sulphate sulphatase, glucoronate 2-sulphate sulphatase, steryl-sulfatase, and extracellular sulfatase Sul-1.
25. The method according to item 24, wherein said sulfohydrolase is sulfamidase.
26. The method according to item 25, wherein said sulfamidase is a modified sulfamidase.
27. The method according to any one of the preceding items, wherein said substrate is selected from the group consisting of a natural substrate and a synthetic substrate.
28. The method according to item 27, wherein said substrate is selected from heparin, heparan sulphate, dermatan sulphate, and a functional equivalent thereof.
29. The method according to item 27, wherein said substrate is selected from the non-limiting group of non-reducing end saccharide-sulfonates (R•SO3⁻), non-reducing end saccharide-sulfamates (RN•SO₃⁻), 4-methylumbelliferyl α-L-idopyranosiduronic acid 2-sulphate (MU-αldoAS), 4-methylumbelliferyl α-D-N-sulphoglucosaminide (MU-αGlcNS), 4-methylumbelliferyl β-D-galactopyranoside-6-sulphate (MU-βGal6S), sulfatide, cerebroside sulfate, oligosaccharides of heparin and heparan sulfate, fondaparinux fragments and heparin disaccharide I-S.
30. The method according to anyone of the preceding items, wherein said substrate is 4-methylumbelliferyl-α-D-N-sulphoglucosaminide (MU-αGlcNS).
31. Use of a barium-rhodizonate complex in solution to determine enzymatic activity of a sulfohydrolase, said complex being formed by combination of a methanol solution comprising barium with a solution comprising alkali metal rhodizonate in at least one solvent selected from the group of alcohols with 2-6 carbons, ether alcohols with 4-6 carbons, heterocycles with at least one heteroatom and polar aprotic solvents.
32. Use of a barium-rhodizonate complex in solution according to item 31, wherein the alcohol with 2-6 carbons is selected from ethanol, propanol, 2-propanol, 2-butanol, *tert*-butanol, 1,3-propanediol, 1,4-butanediol, 1,3-butanediol, 1,5-pentanediol, 3-methyl-1,3-butanediol, 2-methyl-2,4-pentanediol, 1,6-hexanediol.
33. Use of a barium-rhodizonate complex in solution according to item 31, wherein the ether alcohol with 4-6 carbons is selected from 3-methoxy-1-propanol, 2-propoxyethanol, 2-butoxyethanol and 1-methoxy-2-propanol.
34. Use of a barium-rhodizonate complex in solution according to item 31, wherein the heterocycle with at least one heteroatom is selected from tetrahydrofuran, furfuryl alcohol, 1,4-dioxane, N-methyl-2-pyrrolidone, pyridine, pyrazine, benzoic acid, benzylamine and caffeine.
35. Use of a barium-rhodizonate complex in solution according to item 31, wherein the polar aprotic solvent is selected from acetone, acetonitrile, urea, dimethylformamide, dimethyl sulfoxide and dimethyl sulfone.
36. Use of a barium-rhodizonate complex in solution according to any one of items 31-35, wherein said sulfohydrolase is a sulfatase.
37. Use of a barium-rhodizonate complex in solution according to item 36, wherein said sulfatase is a modified sulfatase.
38. Method of analyzing an amount of sulphate ions in a sample, said method comprising a step of colorimetric analysis, comprising colorimetrically measuring an amount of a barium-rhodizonate complex in the presence of any sulphate ions in said sample, said amount of barium-rhodizonate complex being proportional to the amount of sulphate ions in said sample, wherein said barium-rhodizonate complex during said step of measuring is present in a solution comprising methanol and at least one solvent selected from the group of an alcohol with 2-6 carbons, an ether alcohol with 4-6 carbons, a heterocycle with at least one heteroatom and a polar aprotic solvent.
39. Method according to item 38, wherein said sample is selected from an organic and inorganic sample.
40. Method according to item 38, wherein said sample is an aqueous sample, such as a sample selected from water, a beverage, a biological fluid, a growth media and sewage.

## Claims

1. A method of analyzing the enzymatic activity of a sulfohydrolase, comprising a step of colorimetric analysis, said colorimetric analysis comprising:
colorimetrically measuring the amount of a barium-rhodizonate complex in the presence of any sulphate ions that have been enzymatically removed from a substrate by said sulfohydrolase, said substrate containing at least one sulphur-heteroatom bond and said amount of barium-rhodizonate complex being inversely proportional to the enzymatic activity of said sulfohydrolase,
wherein said barium-rhodizonate complex during said step of measuring is present in a solution comprising methanol and at least one solvent selected from the group of an alcohol with 2-6 carbons, an ether alcohol with 4-6 carbons, a heterocycle with at least one heteroatom and a polar aprotic solvent.

2. A method of manufacturing a sulfohydrolase, comprising expressing a sulfohydrolase in a cell culture, and
analyzing the enzymatic activity of said sulfohydrolase by colorimetric analysis.

3. The method of manufacturing a sulfohydrolase according to claim 2, wherein said colorimetric analysis comprises use of a barium-rhodizonate complex in solution.

4. The method of manufacturing a sulfohydrolase according to claim 3, wherein said use comprises measuring an amount of a barium-rhodizonate complex in solution in the presence of any sulphate ions that have been enzymatically removed from a substrate by said sulfohydrolase, said substrate containing at least one sulphur-heteroatom bond.

5. The method of manufacturing a sulfohydrolase according to any one of claims 2 to 4, wherein the enzymatic activity of said produced sulfohydrolase is colorimetrically measured according to the method of claim 1.

6. The method according to any one of the preceding claims, wherein said colorimetric analysis further comprises contacting said sulfohydrolase with said substrate, thereby allowing enzymatic removal of sulphate ions from said substrate.

7. The method according to any one of the preceding claims, wherein said any sulphate ions forms barium-sulphate with barium, whereby a reduction of barium-rhodizinate complex in solution is provided.

8. The method according to any one of the preceding claims wherein the amount of barium-rhodizonate is measured spectrophotometrically, optionally at a wavelength of 480-520 nm, such as 500-520 nm, such as 510 nm.

9. The method according to any one of the preceding claims, wherein methanol and said at least one solvent is present in said solution in a ratio of between 8:2 to 6:4 (v/v), such as 7:3 (v/v).

10. The method according to any one of the preceding claims, wherein the ratio (v/v) of solvent to methanol is from 0.1 to 1, preferably from 0.2 to 0.4.

11. The method according to any one of the preceding claims, wherein the alcohol with 2-6 carbons is selected from ethanol, propanol, 2-propanol, 2-butanol, tert-butanol, 1,3-propanediol, 1,4-butanediol, 1,3-butanediol, 1,5-pentanediol, 3-methyl-1,3-butanediol, 2-methyl-2,4-pentanediol, 1,6-hexanediol; the ether alcohol with 4-6 carbons is selected from 3-methoxy-1-propanol, 2-propoxyethanol, 2-butoxyethanol and 1-methoxy-2-propanol; the heterocycle with at least one heteroatom is selected from tetrahydrofuran, furfuryl alcohol, 1,4-dioxane, N-methyl-2-pyrrolidone, pyridine, pyrazine, benzoic acid, benzylamine and caffeine, and the polar aprotic solvent is selected from acetone, acetonitrile, urea, dimethylformamide, dimethyl sulfoxide and dimethyl sulfone.

12. The method according to any one of the preceding claims, further comprising the steps of:
a) contacting said sulfohydrolase with said substrate, thereby allowing enzymatic removal of sulphate from said substrate and forming a mixture comprising sulfohydrolase, substrate and any sulphate being removed from said substrate;
b) providing a methanol solution comprising barium to said mixture of a), thereby allowing formation of barium-sulphate from barium and said any sulphate, and forming a mixture comprising sulfohydrolase, substrate, any barium-sulphate and remaining barium, and
c) providing a solvent comprising an alkali metal rhodizonate to the mixture of b), thereby forming barium-rhodizonate with said remaining barium.

13. The method according to any one of the preceding claims, wherein said sulfohydrolase is selected from the group consisting of sulfamidase, iduronate 2-sulfatase, arylsulfatase A, arylsulfatase B, arylsulfatase E, arylsulfatase F, arylsulfatase G, arylsulfatase H, arylsulfatase I, arylsulfatase J, arylsulfatase K, N-acetylgalactosamine-4-sulfatase, N-acetylgalactosamine-6-sulfatase, N-acetylglucosamine-6-sulfatase, N-sulphoglucosamin sulphohydrolase, glucosamine 3-sulphate sulphatase, glucoronate 2-sulphate sulphatase, steryl-sulfatase, and extracellular sulfatase Sul-1.

14. Use of a barium-rhodizonate complex in solution to determine enzymatic activity of sulfohydrolase, said complex being formed by combination of a methanol solution comprising barium with a solution comprising alkali metal rhodizonate and at least one solvent selected from the group of monohydric alcohols with 2-4 carbons, ether alcohols with 4-6 carbons, heterocycle with at least one heteroatom and the group of polar aprotic solvents.
